(19) Europäisches Patentamt
European Patent Office
Office européen des brevets

(11) EP 0 842 136 B1

(12) **FASCICULE DE BREVET EUROPEEN**

(45) Date de publication et mention de la délivrance du brevet:
**20.10.1999 Bulletin 1999/42**

(21) Numéro de dépôt: **96924928.3**

(22) Date de dépôt: **03.07.1996**

(51) Int Cl.[6]: **C07C 19/075**, C07C 17/16, C07C 17/093, C07C 29/64

(86) Numéro de dépôt international:
**PCT/FR96/01036**

(87) Numéro de publication internationale:
**WO 97/03037 (30.01.1997 Gazette 1997/06)**

(54) **PROCEDE DE PREPARATION D'ALPHA,OMEGA-BROMOCHLOROALCANES**

VERFAHREN ZUR HERSTELLUNG VON ALPHA,OMEGA-BROMOCHLOROALKANEN

METHOD FOR PREPARING ALPHA,OMEGA-BROMOCHLOROALKANES

(84) Etats contractants désignés:
**AT BE CH DE DK ES FR GB IE IT LI NL**

(30) Priorité: **11.07.1995 FR 9508361**

(43) Date de publication de la demande:
**20.05.1998 Bulletin 1998/21**

(73) Titulaire: **ELF ATOCHEM S.A.**
**92800 Puteaux, Hauts-de-Seine (FR)**

(72) Inventeurs:
- **DRIVON, Gilles**
**F-69850 Saint-Martin-en-Haut (FR)**
- **RUPPIN, Christophe**
**F-69310 Pierre-Bénite (FR)**

(56) Documents cités:
**US-A- 2 218 018** **US-A- 3 206 516**

- **JOURNAL OF ORGANIC CHEMISTRY, vol. 59, EASTON US, pages 2616-2619, XP002015943 M.A. FORTH ET AL.: "Imine-Directed Metalation of o-Tolualdehyde: The Use of Catalytic Amine Base. A Route to 2(8-Phenyloctyl)benzaldehyde"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 842 136 B1

## Description

La présente invention concerne un procédé de préparation directe d'$\alpha$,$\omega$-bromochloroalcanes à partir d'ethers-oxydes (éthers) cycliques.

Les halogénoalcanes et, plus particulièrement les $\alpha$,$\omega$-bromochloroalcanes sont largement utilisés comme réactifs de départ pour la préparation de produits pharmaceutiques, de pesticides et de détergents.

De nombreuses méthodes ont été décrites pour obtenir ces $\alpha$,$\omega$-bromochloroalcanes.

Le plus souvent, elles font appel à la réaction des halogènes (brome, chlore) ou de leurs dérivés tels que $PBr_3$, $SBr_6$ sur un $\alpha$, $\omega$-chlorohydroxyalcane ou encore à la réaction des halogènes (brome, chlore) ou de leurs dérivés tels que $SOCl_2$ sur un halogénoalcane ou un acide $\omega$-halogènoalcanoïque.

Le brevet britannique 788,349 décrit un procédé de préparation de 1-bromo-4-chlorobutane qui consiste à traiter dans une première étape le THF avec de l'acide chlorhydrique sec en présence de traces de $ZnCl_2$ à une température qui atteint environ 100°C, puis dans une seconde étape à traiter le 4-chloro-1-butanol obtenu précédemment avec du phosphore rouge puis avec du brome sec à une température comprise entre 0°C et -10°C. Le 1-bromo-4-chlorobutane est obtenu avec un rendement d'environ 62 % par rapport au THF mis en oeuvre.

Le brevet US 2,839,574 mentionne un procédé de préparation de 1-bromo-4-chlorobutane qui évite l'emploi de phosphore rouge et de brome ou de $SBr_6$. Ce procédé consiste à traiter du 4-chloro-1-butanol préalablement distillé avec de l'acide bromhydrique gazeux et sec en présence d'un solvant à ébullition et formant un azéotrope avec l'eau formée selon la réaction :

$$Cl(CH_2)_4OH + HBr \rightarrow Br\ (CH_2)_4Cl + H_20.$$

Le rendement est d'environ 70 %.

Dans la demande de brevet Japonais publiée sous le No JP 5791930, le 1-bromo-4-chlorobutane a été obtenu avec un rendement d'environ 90 % en traitant le 4-chloro-1-butanol fraîchement distillé avec $SBr_6$ formé à partir de soufre et de brome, selon le schéma réactionnel :

$$SBr_6 + 6\ Cl(CH_2)_4OH \rightarrow 6\ Br(CH_2)_4Cl + H_2SO_4 + 2H_2O$$

Dans le cas où l'on fait réagir $SBr_6$ avec un 4-chloro-1-butanol non purifié, provenant notamment d'un mélange de tétrahydrofurane et d'acide chlorhydrique, le rendement en 1-bromo-4-chlorobutane est d'environ 70 %.

D.C. SAYLES et Ed. F. DEGERING (Journal of American Chemistry Society, 71, page 3162, 1949) décrivent une méthode de préparation du 1-bromo-4-chlorobutane en traitant le n-bromobutane avec du chlorure de sulfuryle ($SO_2Cl_2$) en présence de peroxyde de benzoyle, au reflux des réactifs. Le rendement en 1-bromo-4-chlorobutane est de 35 %.

SMUSHKEVICH, YU.I et coll. appliquent la réaction de BORODINE-HUNSDIECKER aux acides $\omega$-chloroalcanoiques (Tr. Mosk. Khim. Technol. Inst. No 61, pp 47-48, 1969).

Ils obtiennent ainsi des $\alpha$,$\omega$-bromochloroalcanes en traitant les acides $\omega$-chloroalcanoiques avec HgO et du brome en milieu $CCl_4$ selon le schéma réactionnel :

$$Cl(CH_2)_n\ COOH + Br_2 \rightarrow Cl(CH_2)_n\ Br + CO_2 + HBr$$

Toutes ces méthodes présentent de nombreux inconvénients. Elles utilisent des composés de départ souvent impurs nécessitant des opérations de purification ainsi que des réactifs coûteux et de manipulation difficile (P + brome , S + brome).

Les rendements en $\alpha$,$\omega$-bromochloroalcanes sont faibles et les produits obtenus contiennent des impuretés dont l'élimination entraîne des manipulations de séparation difficiles et coûteuses.

On a maintenant trouvé un procédé de préparation directe d'$\alpha$,$\omega$-bromochloroalcanes de formule

$Br(CH_2)_n\ Cl$ (I)dans laquelle n représente un nombre entier allant de 3 à 8, à partir d'éther-oxyde cyclique de formule

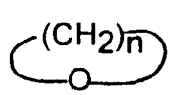

(II)

dans laquelle n a la même signification que dans la formule (I), caractérisé en ce que :

a/ l'on met en contact ledit éther-oxyde cyclique (II), avec de l'acide bromhydrique gazeux, puis
b/ l'on met en contact la phase précédente obtenue en a/ avec du chlorure de thionyle ($SOCl_2$) et un composé renfermant un groupe amide d'acide carboxylique N-alkylé ou N-dialkylé.

A titre d'exemple d'éther-oxyde cyclique de formule (II) utilisable selon la présente invention, on peut citer l'oxyde de 1,3-propylène (oxétane), le tétrahydrofurane, le tétrahydropyrane, l'oxyde de 1,6-hexaméthylène(oxépane), l'oxyde de 1,7-heptaméthylène (oxocane).
Le procédé de la présente invention s'applique tout particulièrement à la préparation du 1-bromo-4-chlorobutane à partir du tétrahydrofurane.
A titre d'illustration de composés renfermant un groupé amide d'acide carboxylique N-alkylé ou N-dialkylé utilisables selon la présente invention, on citera le N-méthylacétamide, le diméthylformamide, le diéthylformamide. Parmi ces composés, on préfère utiliser le diméthylformamide.
On utilisera des quantités pondérales de ces composés au plus égales à 5 % par rapport à l'éther oxyde mis en oeuvre et, de préférence, des quantités pondérales comprises entre 0,1 % et 3 %.
Selon la présente invention, il n'est pas nécessaire d'isoler et/ou de purifier l'α,ω-bromohydroxyalcane (III) obtenu en a/ par hydrobromation de l'éther-oxyde cyclique (II) selon le schéma réactionnel :

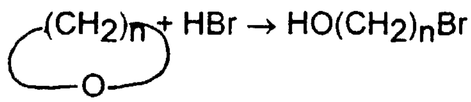 (A)

(II) (III)

L'étape b/ est effectuée en mettant en contact directement la phase précédente obtenue en a/, qui contient l'intermédiaire (III) avec du chlorure de thionyle et un composé renfermant un groupe amide d'acide carboxylique N-alkylé ou N-dialkylé pour conduire à l'α,ω-bromochloroalcane (I) selon le schéma réactionnel :

$$HO(CH_2)_nBr + SOCl_2 \rightarrow Br(CH_2)_nCl + HCl + SO_2 \quad (B)$$

(III) (I)

L'un des avantages de l'invention apparaît clairement ici ; il n'est pas nécessaire d'isoler et/ou de purifier l'α, ω-bromohydroxyalcane (III) formé intermédiairement dans l'étape a/ avant de le mettre en contact avec le chlorure de thionyle (étape b/).
Le procédé selon l'invention peut être réalisé à des températures allant d'environ 0°C à 30°C pour l'étape a/ et d'environ 20°C à 130°C et, de préférence de 50°C à 70°C pour l'étape b/.
Une température supérieure à 30°C dans l'étape a/ serait de nature à abaisser la teneur en composé intermédiaire (III) et à conduire à la formation de produits secondaires indésirables tels que des dibromoalcanes.
On opère avec un rapport HBr / éther-oxyde cyclique quasiment égal à 1 et un rapport $SOCl_2$/éther-oxyde cyclique compris entre 0,90 et 1,50 et, de préférence, compris entre 0,95 et 1,20.
La durée des étapes a/ et b/ peut varier dans de larges limites, mais est généralement comprise entre 5 heures et 20 heures et, de préférence, comprise entre 10 et 15 heures.
La réaction peut être effectuée sous pression atmosphérique. Selon une façon d'opérer, on introduit sous agitation l'acide bromhydrique gazeux dans l'éther-oxyde cyclique. L'introduction terminée, on dégaze éventuellement l'acide bromhydrique non réagi du milieu réactionnel sous pression réduite ou par entraînement avec un gaz inerte tel que l'azote, et, de préférence à température ambiante. Puis, après avoir introduit un composé renfermant un groupe amide d'acide carboxylique N-alkylé ou N-dialkylé, on ajoute du chlorure de thionyle.
L'acide chlorhydrique et le dioxyde de soufre formés dégazent en continu du milieu réactionnel.
Ce dégazage peut être favorisé, notamment en fin de réaction, par un balayage avec un gaz inerte ou bien en mettant le milieu réactionnel sous pression réduite.
L'acide chlorhydrique et le dioxyde de soufre éliminés peuvent être ensuite neutralisés dans une ou plusieurs colonnes d'absorption contenant de la soude et un sulfite alcalin comme par exemple du sulfite de sodium.
On ne sortirait pas du cadre de l'invention si l'on effectuait l'étape b/ en absence de composé renfermant un groupe amide d'acide carboxylique N-alkylé ou N-dialkylé.

La préparation d'α,ω-bromochloroalcanes selon le procédé de l'invention présente l'avantage d'être effectué en deux étapes réactionnelles successives, dans un même appareillage, sans une quelconque purification ou séparation de l'α,ω-bromohydroxyalcane intermédiaire.
Un autre avantage du procédé selon l'invention est d'être effectué sans solvant.
Les rendements en α,ω-bromochloroalcanes sont élevés et les produits obtenus sont purifiés par des méthodes connues de l'homme du métier telle que la distillation.
Les exemples qui suivent illustrent l'invention .

## Exemple 1

Etape a

On introduit dans un réacteur de 1 litre, surmonté d'une colonne à garnissage d'anneaux de Raschig et d'un condenseur refroidi à -25°C muni d'une agitation, d'une prise de température et d'une enveloppe dans laquelle circule un fluide thermique, 216 g de tetrahydrofurane (THF) soit 3 moles à température ambiante, puis sous agitation, on injecte en pied de réacteur de l'acide bromhydrique gazeux à un débit de 1 mol/h pendant 2 heures puis ensuite à un débit de 0,5 mol/h pendant 2 heures, tout en maintenant la température du milieu réactionnel à la température ambiante (environ 20°C).
L'analyse par chromatographie en phase gazeuse (CPG) de la phase réactionnelle obtenue révèle qu'elle contient 91 % en poids de 4-bromo-1-butanol, 5 % en poids de 1,4-dibromobutane et 4 % en poids de THF non transformé.
Le rendement molaire brut en 4-bromo-1-butanol est égal à 88 % par rapport au THF mis en oeuvre.

Etape b

A la phase a/ obtenue précédemment, on introduit 0,84 g de diméthylformamide. On porte le milieu réactionnel à 50°C puis on coule 357 g de $SOCl_2$, soit 3 moles, sous agitation en 90 minutes tout en maintenant la température à 50°C. L'addition de $SOCl_2$ terminée, on élève la température du milieu réactionnel à 70°C, température que l'on maintient pendant 2 heures l'acide chlorhydrique et le $SO_2$ formés dégazant en continu sont neutralisés au moyen d'une colonne d'absorption contenant un mélange de soude et de sulfite de sodium.
Après refroidissement à température ambiante, le milieu réactionnel est lavé avec 105 g d'eau puis décanté. On obtient 504 g d'une phase organique contenant 84,7 % en poids de 1-bromo-4-chlorobutane, 9,3 % en poids de 1,4-dibromobutane, 2,5 % en poids de 1,4-dichlorobutane et 1,9 % de THF, soit un rendement molaire brut en 1-bromo-4-chlorobutane de 83 % par rapport au THF mis en oeuvre.
La phase organique est soumise à une distillation sous une pression réduite de 25 mmHg dans une colonne adiabatique de 30 plateaux théoriques.
On obtient un 1-bromo-4-chlorobutane ($Eb_{25mmHg}$ = 76°C/76,5°C) avec une pureté supérieure à 99,5 %. Le rendement de distillation est de l'ordre de 75 %.

## Exemple 2

Les conditions opératoires des étapes a/ et b/ sont identiques à celles des étapes a/ et b/ de l'exemple 1, excepté que l'on coule le $SOCl_2$ à 70°C au lieu de 50°C.
On obtient un milieu réactionnel (avant purification) qui est constitué de :

73,2 % en poids de 1-bromo-4-chlorobutane,
15,7 % en poids de 1,4-dibromobutane,
6 % en poids de 1,4-dichlorobutane,
2,4 % en poids de THF.

On obtient un rendement molaire brut de 1-bromo-4-chlorobutane de 70,5 % par rapport au THF mis en oeuvre. Le milieu réactionnel est traité comme dans l'exemple 1 et la phase organique est soumise à une distillation sous pression réduite.

## Exemple 3

On opère comme dans l'exemple 2, excepté que dans l'étape b/, le $SOCl_2$ est ajouté à une température de 60°C au lieu de 70°C.

On obtient un milieu réactionnel (avant purification) qui est constitué de :

81 % en poids de 1-bromo-4-chlorobutane,
11,5 % en poids de 1,4-dibromobutane,
4 % en poids de 1,4-dichlorobutane,
1,3 % en poids de THF.

On obtient un rendement molaire brut en 1-bromo-4-chlorobutane de 78 %.

**Exemple 4** - Non conforme à l'invention

L'étape a/ est réalisée avec des quantités de réactifs identiques à l'exemple 1, mais selon des conditions opératoires différentes. Au début de l'injection de HBr la température est de 67°C et en fin d'injection de HBr, la température est de 105°C.

L'analyse par CPG de la phase réactionnelle révèle qu'elle contient 52 % en poids de 4-bromo-1-butanol, 36 % en poids de 1,4-dibromobutane et 12 % en poids de THF. Ceci correspond à un rendement molaire en 4-bromo-1-butanol d'environ 50 % par rapport au THF mis en oeuvre.

**Exemple 5**

Etape a/

Cette étape a/ est réalisée selon des conditions opératoires identiques à celles de l'étape a/ de l'exemple 1, excepté que l'on utilise 8 moles de THF et 8 moles de HBr qui sont introduits à un débit de 1,5 mol/h pendant 5 heures puis à un débit de 1 mol/h pendant 30 minutes.

Etape b/

On coule directement 9,5 moles de $SOCl_2$ (excès molaire de 18,7 % par rapport au THF mis en oeuvre) à 50°C pendant 4 heures, puis on chauffe 2h30 à 65°C-85°C et 2 heures à 130°C.

Après refroidissement, on lave à l'eau le milieu réactionnel et on décante.

L'analyse par CPG de la phase organique décantée révèle que l'on a obtenu le 1-bromo-4-chlorobutane avec un rendement molaire brut de 77,5 % par rapport au THF mis en oeuvre.

## Revendications

1. Procédé de préparation directe d'α,ω-bromochloroalcanes de formule

$$Br(CH_2)_nCl \quad (I)$$

dans laquelle n représente un nombre entier allant de 3 à 8 à partir d'éther-oxyde cyclique de formule

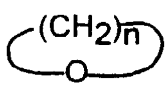

(II)

dans laquelle n a la même signification que dans la formule (I), caractérisé en ce que :

a/ l'on met en contact ledit éther-oxyde cyclique (II) avec de l'acide bromhydrique gazeux, puis
b/ l'on met en contact la phase précédente obtenue en a/ avec du chlorure de thionyle ($SOCl_2$) et un composé renfermant un groupe amide d'acide carboxylique N-alkylé ou N-dialkylé.

2. Procédé selon la revendication 1, caractérisé en ce que l'éther-oxyde cyclique (II) est le tétrahydrofurane.

3. Procédé selon la revendication 1, caractérisé en ce que le composé renfermant un groupe amide d'acide carboxylique N-dialkylé est le diméthylformamide.

**4.** Procédé selon l'une des revendications 1 et 3, caractérisé en ce que le composé renfermant un groupe amide d'acide-carboxylique N-alkylé ou N-dialkylé est utilisé en une quantité pondérale au plus égale à 5 % par rapport à l'éther-oxyde cyclique et, de préférence, une quantité pondérale comprise entre 0,1 % et 3 %.

**5.** Procédé selon l'une des revendications 1 à 4, caractérisé en ce que le rapport molaire acide bromhydrique gazeux/éther-oxyde cyclique est quasiment égal à 1.

**6.** Procédé selon l'une des revendications 1 à 4, caractérisé en ce que le rapport molaire chlorure de thionyle/éther-oxyde cyclique est compris entre 0,90 et 1,50.

**7.** Procédé selon la revendication 6, caractérisé en ce que le rapport molaire chlorure de thionyle/éther-oxyde cyclique est compris entre 0,95 et 1,20.

**8.** Procédé selon l'une des revendications 1 à 7 caractérisé en ce que la température de l'étape a/ va de 0°C à 30°C.

**9.** Procédé selon l'une des revendications 1 à 7, caractérisé en ce que la température de l'étape b/ va de 20°C à 130°C.

**10.** Procédé selon la revendication 9, caractérisé en ce que la température de l'étape b/ va de 50°C à 70°C.

**Patentansprüche**

**1.** Verfahren zur direkten Herstellung von α,ω-Bromchloralkanen der Formel

$$Br(CH_2)_nCl \qquad (I)$$

in der n eine ganze Zahl von 3 bis 8 bedeutet, aus cyclischem Ether der Formel

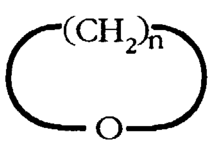

(II)

in der n die gleiche Bedeutung wie in Formel (I) hat, dadurch gekennzeichnet, daß:

a) man den cyclischen Ether (II) mit gasförmigem Bromwasserstoff zusammengibt und
b) man die zuvor in a) erhaltene Phase mit Thionylchlorid ($SOCl_2$) und einer Verbindung zusammengibt, die eine N-Alkyl- oder N-Dialkylcarbonsäureamid-Gruppe enthält.

**2.** Verfahren nach Anspruch 1, dadurch gekennzeichnet, daß der cyclische Ether (II) Tetrahydrofuran ist.

**3.** Verfahren nach Anspruch 1, dadurch gekennzeichnet, daß die Verbindung, die eine N-Dialkylcarbonsäureamid-Gruppe enthält, Dimethylformamid ist.

**4.** Verfahren nach einem der Ansprüche 1 und 3, dadurch gekennzeichnet, daß die Verbindung, die eine N-Alkyl- oder N-Dialkylcarbonsäureamid-Gruppe enthält, in einer Gewichtsmenge von höchstens 5 %, bezogen auf den cyclischen Ether, vorzugsweise in einer Gewichtsmenge zwischen 0,1 % und 3 % verwendet wird.

**5.** Verfahren nach einem der Ansprüche 1 bis 4, dadurch gekennzeichnet, daß das Molverhältnis von gasförmigem Bromwasserstoff zum cyclischen Ether ungefähr 1 beträgt.

**6.** Verfahren nach einem der Ansprüche 1 bis 4, dadurch gekennzeichnet, daß das Molverhältnis von Thionylchlorid zum cyclischen Ether zwischen 0,9 und 1,5 liegt.

**7.** Verfahren nach Anspruch 6, dadurch gekennzeichnet, daß das Molverhältnis von Thionylchlorid zum cyclischen Ether zwischen 0,95 und 1,20 liegt.

**8.** Verfahren nach einem der Ansprüche 1 bis 7, dadurch gekennzeichnet, daß die Temperatur in Schritt a) zwischen 0 °C und 30 °C liegt.

**9.** Verfahren nach einem der Ansprüche 1 bis 7, dadurch gekennzeichnet, daß die Temperatur in Schritt b) zwischen 20 °C und 130 °C liegt.

**10.** Verfahren nach Anspruch 9, dadurch gekennzeichnet, daß die Temperatur in Schritt b) zwischen 50 °C und 70 °C liegt.

**Claims**

**1.** Process for the direct preparation of α,ω-bromochloroalkanes of formula

$$Br(CH_2)_nCl \qquad (I)$$

in which n represents an integer ranging from 3 to 8, starting with a cyclic oxide ether of formula

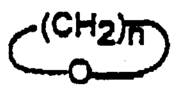

(II)

in which n has the same meaning as in formula (I), characterized in that:

a/ the said cyclic oxide ether (II) is placed in contact with gaseous hydrogen bromide, and then
b/ the phase obtained in a/ above is placed in contact with thionyl chloride ($SOCl_2$) and a compound containing an N-alkyl or N-dialkyl carboxamide,

**2.** Process according to Claim 1, characterized in that the cyclic oxide ether (II) is tetrahydrofuran.

**3.** Process according to Claim 1, characterized in that the compound containing an N-dialkyl carboxamide group is dimethylformamide.

**4.** Process according to either of Claims 1 and 3, characterized in that the compound containing an N-alkyl or N-dialkyl carboxamide group is used in an amount by weight of not more than 5% relative to the cyclic oxide ether and preferably in an amount by weight of between 0.1% and 3%.

**5.** Process according to one of Claims 1 to 4, characterized in that the gaseous hydrogen bromide/cyclic oxide ether molar ratio is virtually equal to 1.

**6.** Process according to one of Claims 1 to 4, characterized in that the thionyl chloride/cyclic oxide ether molar ratio is between 0.90 and 1.50.

**7.** Process according to Claim 6, characterized in that the thionyl chloride/cyclic oxide ether molar ratio is between 0.95 and 1.20.

**8.** Process according to one of Claims 1 to 7, characterized in that the temperature of step a/ ranges from 0°C to 30°C.

**9.** Process according to one of Claims 1 to 7, characterized in that the temperature of step b/ ranges from 20°C to

130°C.

**10.** Process according to Claim 9, characterized in that the temperature of step b/ ranges from 50°C to 70°C.